# FASCICULE DE BREVET EUROPEEN

(11) **EP 3 145 578 B1**
(45) Date de publication et mention de la délivrance du brevet: **03.08.2022**
(21) Numéro de dépôt: 15730279.5
(22) Date de dépôt: 19.05.2015
(51) Int. Cl.: A61N 1/04, A61N 1/32, A61N 1/36

(54) **DISPOSITIF MÉDICAL CUTANÉ COMPRENANT UNE PARTIE PRINCIPALE AVEC UNE EMBASE ET UNE ÉLECTRODE AMOVIBLE**
MEDIZINISCHES GERÄT FÜR DIE HAUT UMFASSEND EINEN HAUPTTEIL MIT EINER BASIS UND EINE ABNEHMBARE ELEKTRODE
CUTANEOUS MEDICAL DEVICE COMPRISING A PRINCIPAL PART WITH A BASE AND A REMOVABLE ELECTRODE

(30) Priorité: 19.05.2014 FR 1454456
(43) Date de publication de la demande: 29.03.2017
(73) Titulaire: Commissariat à l'Énergie Atomique et aux Énergies Alternatives, 75015 Paris (FR)
(72) Inventeur: PERRAUD, Simon, F-83150 Bandol (FR); KARST, Nicolas, F-57600 Folkling (FR)
(74) Mandataire: Gevers & Orès
(86) Numéro de dépôt international: PCT/IB2015/053680
(87) Numéro de publication internationale: WO 2015/177725

(56) Documents cités:
- EP-A1- 1 106 204
- WO-A2-2013/107635
- FR-A1- 2 961 340
- US-A- 5 562 607
- US-A1- 2012 253 263
- US-A1- 2013 046 212
- US-A1- 2013 281 914

## Description

L'invention concerne le domaine technique des dispositifs destinés à être fixés sur la peau d'un utilisateur.

Il s'agit notamment de dispositifs médicaux, comme des générateurs d'impulsions pour l'électrostimulation.

On rappelle ici que l'électrostimulation est une technique consistant à stimuler électriquement les nerfs ou les muscles. L'électrostimulation des nerfs (notamment la technique d'électrostimulation transcutanée des nerfs, ou transcutaneous electrical nerve stimulation (TENS) dans la terminologie anglaise) permet notamment de traiter la douleur. L'électrostimulation des muscles (neuromuscular electrical stimulation (NMES) dans la terminologie anglaise) peut être utilisée à des fins de rééducation, récupération musculaire ou renforcement musculaire.

Les dispositifs d'électrostimulation comprennent un générateur d'impulsions électriques relié à des électrodes cutanées. Le générateur d'impulsions permet d'envoyer des impulsions électriques calibrées en fréquence et en intensité jusqu'à une zone précise du corps humain par l'intermédiaire des électrodes cutanées. Dans les dispositifs conventionnels, le générateur d'impulsions se présente sous la forme d'un boitier volumineux et rigide.

A titre d'exemple, un générateur d'impulsions tel que décrit dans le document Mark Johnson, Transcutaneous Electrical Nerve Stimulation (Johnson, Mark I (October 2012), Transcutaneous Electrical Nerve Stimulation (TENS), eLS. John Wiley & Sons, Ltd: Chichester) se présente sous la forme d'un boitier de volume de l'ordre de plusieurs dizaines de centimètres cube et d'épaisseur de l'ordre de quelques centimètres.

Ce type de générateur d'impulsions n'est pas pratique d'utilisation. En effet, il s'agit d'un objet encombrant qui est relié aux électrodes cutanées par l'intermédiaire de câbles électriques relativement longs (typiquement de longueur supérieure à 1 m).

Il a été proposé dans l'art antérieur de remplacer les générateurs d'impulsions conventionnels, volumineux et rigides, par des générateurs d'impulsions fins qui peuvent être directement portés par l'utilisateur sous la forme de patchs.

Un tel générateur d'impulsions, fin et flexible, se présentant sous la forme d'un patch, est décrit dans le document US 5,562,607 et dans le document US-5,423,874.

Dans ce patch, sont assemblés, sur un circuit flexible, la source d'énergie du type batterie et les composants électroniques qui sont des composants discrets et des circuits intégrés permettant de faire fonctionner le générateur d'impulsions.

Le circuit flexible est encapsulé entre une couche supérieure imperméable et une couche inférieure adhésive. La couche supérieure imperméable joue le rôle de barrière à l'humidité pour protéger la source d'énergie et les composants électroniques. La couche inférieure adhésive, qui comprend deux électrodes cutanées, permet de fixer le générateur d'impulsions sur la peau.

Ce générateur d'impulsions est moins encombrant que les générateurs d'impulsions conventionnels et il permet de réduire considérablement la longueur des câbles électriques, voire de se passer complètement de ces câbles.

Un dispositif d'électrosimulation peut comporter plusieurs électrodes, par exemple une ou plusieurs électrodes fixes et une ou plusieurs électrodes amovibles, ou encore plusieurs électrodes amovibles.

Le nombre et le type d'électrodes fixes sont connus du dispositif. Ce n'est pas le cas pour les électrodes amovibles.

Or, un dispositif d'électrosimulation peut mettre en oeuvre différents programmes, lesquels peuvent nécessiter des types et et un nombre particulier d'électrodes amovibles.

Dans la mesure où les électrodes amovibles sont fixées par l'utilisateur, il est nécessaire de déterminer quelles sont les électrodes connectées pour s'assurer que le programme choisi peut effectivement être lancé.

Or, aucun dispositif d'electrostimulation connu ne fournit de solutions techniques à ce problème.

De plus, un dispositif d'électrostimulation tel que décrit dans le document US-5,423,874 n'est pas polyvalent : la distance inter-électrodes est fixe et donc ne peut pas être ajustée par le patient en fonction de sa pathologie et de sa morphologie.

L'invention a pour objet de pallier ces inconvénients en proposant un dispositif médical cutané, notamment un dispositif d'électrostimulation destiné à être fixé sur la peau d'un utilisateur, qui permette la reconnaissance d'électrodes cutanées amovibles et qui offre une grande liberté d'ajustement de la distance inter-électrodes.

Ainsi, l'invention concerne un dispositif médical cutané selon la revendication 1.

Selon l'invention, le dispositif comprend, dans ladite au moins une embase, des moyens conducteurs et, dans ladite fiche, un moyen de contrôle du passage du courant entre lesdits moyens conducteurs, lesdits moyens conducteurs et ledit moyen de contrôle formant un interrupteur électrique ayant une fonction de reconnaissance de ladite électrode cutanée amovible.

Cette fonction de reconnaissance comporte au moins deux aspects : la possibilité de détecter le type d'électrode cutanée connectée à une embase donnée et celle de détecter les embases dans lesquelles une électrode cutanée a été connectée.

Par ailleurs, l'embase comporte au moins une pièce ayant une fonction électrique et la fiche comporte une pièce cylindrique ayant une fonction électrique, la connexion électrique entre l'embase et la fiche étant assurée par le contact électrique entre les pièces ayant une fonction électrique.

Dans une première variante, une pièce annulaire a une fonction électrique.

Dans une deuxième variante, trois pièces disposées selon un cercle ont une fonction électrique.

De préférence, ces pièces sont montées sur ressort.

De façon préférée, les moyens conducteurs de l'embase comprennent deux ensembles fixes et isolés électriquement l'un de l'autre.

Dans un premier exemple de réalisation, la fiche de ladite au moins une électrode amovible comporte une protubérance présentant une hauteur (h) telle que la protubérance est susceptible de venir en contact avec les deux ensembles fixes.

Dans un deuxième exemple de réalisation, la fiche de ladite au moins une électrode amovible comporte une protubérance présentant une hauteur (h') telle qu'elle ne vient pas en contact avec les deux ensembles fixes, lorsqu'elle est insérée dans la cavité.

Selon l'invention, lesdits moyens conducteurs de l'embase comprennent également une pièce déformable placée au-dessus des deux ensembles.

Dans un premier exemple de réalisation, la fiche de ladite au moins une électrode amovible comporte une protubérance présentant une hauteur (h) telle que la protubérance est susceptible de déformer ladite pièce lorsqu'elle est insérée dans la cavité.

Dans un deuxième exemple de réalisation, la fiche de ladite au moins une électrode amovible comporte une protubérance présentant une hauteur (h') telle qu'aucune pression n'est exercée par la protubérance sur ladite pièce, lorsqu'elle est insérée dans la cavité.

Le dispositif peut comprendre au moins deux embases et au moins deux électrodes cutanées amovibles.

De façon avantageuse, le dispositif comprend au moins deux zones rigides et une zone flexible entre deux zones rigides adjacentes.

Par ailleurs la partie principale peut comporter sur une deuxième face, opposée à la première, une autre électrode cutanée destinée à établir un contact électrique avec l'utilisateur.

De façon préférée, le dispositif comprend des moyens magnétiques à la fois sur la fiche et sur l'embase.

L'invention sera mieux comprise et d'autres buts, avantages et caractéristiques de celle-ci apparaîtront plus clairement à la lecture de la description qui suit et qui est faite au regard des dessins annexés, sur lesquels :
- la figure 1 (1A-1B) comprend une vue de dessus (figure 1A) et une vue en coupe selon la ligne I-I (figure 1B) d'un exemple d'une embase femelle,
- la figure 2 est une vue en coupe d'un dispositif cutané comprenant une embase du type illustré à la figure 1.
- la figure 3 (3A-3B) comprend une vue de dessous (figure 3A) et une vue en coupe selon la ligne III-III (figure 3B) d'une fiche mâle adaptée à l'embase illustrée à la figure 1,
- la figure 4 (4A-4B) comprend une vue de dessus (figure 4A) d'un dispositif cutané et une vue en coupe (figure 4B) d'une fiche mâle adaptée au dispositif cutané illustré à la figure 4A,
- les figures 5 et 6 comprennent des vues de dessus d'un dispositif illustré à la figure 4 dans deux configurations de connexion d'électrodes cutanées amovibles.
- la figure 7 (7A-7B) comprend une vue de dessus (figure 7A) et une vue en coupe selon la ligne VII-VII (figure 7B) d'une variante de réalisation de l'embase illustrée à la figure 1,
- la figure 8 est une vue de dessus d'une variante de réalisation du dispositif cutané illustré à la figure 4A.

Les éléments communs aux différentes figures seront désignés par les mêmes références.

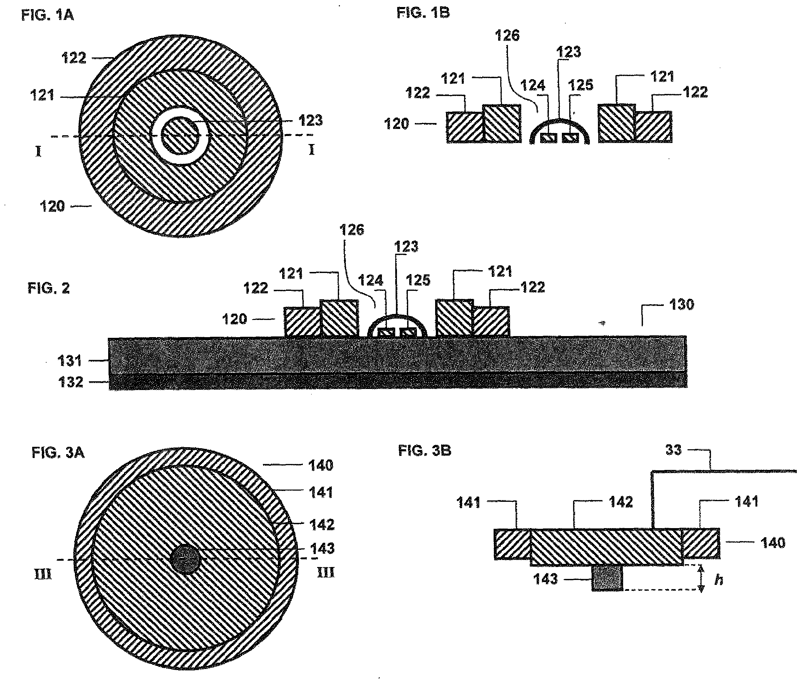

Un mode de réalisation du dispositif selon l'invention va maintenant être décrit en référence aux figures 1 à 6.

La figure 1A (vue de dessus) et la figure 1B (vue en coupe selon la ligne II-II sur la figure 1A) représentent l'embase 120 du connecteur électrique.

Il s'agit d'une embase du type femelle, c'est-à-dire comportant un trou 126.

L'embase 120 comprend deux pièces annulaires 121 et 122 centrées autour d'un même axe, la pièce 121 étant située à l'intérieur de la pièce 122. La pièce 121 a une fonction électrique. Il s'agit par exemple d'une pièce en métal. La pièce 122 a une fonction magnétique. Il s'agit par exemple d'une pièce en ferrite, ou aluminium-nickel-cobalt ou samarium-cobalt ou encore néodyme-fer-bore, qui sont des matériaux aimantés.

Au centre des pièces 121 et 122, est positionné un système comportant notamment une pièce conductrice de l'électricité et déformable 123 en forme de coupole et deux pièces conductrices de l'électricité 124 et 125 en forme de plaques. Les deux pièces 124 et 125 sont isolées électriquement l'une de l'autre. Ces trois pièces peuvent être réalisées en métal.

Avantageusement, seule la face intérieure (tournée vers les pièces 124 et 125) de la coupole 123 est conductrice, sa face extérieure étant isolante.

La figure 2 représente schématiquement un dispositif selon l'invention, ici un patch générateur d'impulsions pour l'électrostimulation 130, intégrant l'embase 120.

Le patch 130 comprend une partie principale 131 qui contient notamment des composants électroniques et une source d'énergie électrique.

Il comprend, sur sa face inférieure, des couches d'hydrogel formant une première électrode cutanée 132, destinée à établir un contact électrique avec la peau de l'utilisateur.

Le patch 130 comprend, sur sa face supérieure, une embase 120. Elle permet notamment de connecter le patch 130 à une seconde électrode cutanée (non représentée sur les figures), via une fiche 140 et un câble électrique 33 (représentés à la figure 3).

Il est maintenant fait référence aux figures 3A (vue de dessous) et 3B (vue en coupe selon la ligne III-III sur la figure 3A) qui représentent la fiche mâle 140 du connecteur électrique, destinée à coopérer avec l'embase 120 illustrée à la figure 1.

La fiche 140 comprend une pièce annulaire 141 et une pièce cylindrique 142 entourée par la pièce annulaire 141 et formant sa base.

La pièce 141 a une fonction magnétique. La pièce 142 a une fonction électrique et elle est reliée à un câble électrique 33, lui-même relié à une électrode cutanée.

La fiche 140 comprend également une pièce cylindrique centrale 143 de hauteur h formant une protubérance, en saillie par rapport aux pièces 141 et 142.

De préférence, la pièce 141 est légèrement en retrait de la pièce 142, du côté de la protubérance 143.

Les trois pièces 141, 142 et 143 sont centrées autour d'un même axe.

La pièce 141 est par exemple une pièce en ferrite, ou aluminium-nickel-cobalt ou samarium-cobalt ou encore néodyme-fer-bore, qui sont des matériaux aimantés.

La pièce 142 est par exemple une pièce en métal.

La pièce 143 est par exemple une pièce en polymère.

Le trou 126 de l'embase 120 est destiné à accueillir la protubérance 143, ce qui empêche une déconnexion accidentelle due à des forces latérales, c'est-à-dire des forces dans le plan de l'embase 120.

La pièce 121 est destinée à établir un contact électrique avec la pièce 142. La pièce 122 et la pièce 141 sont destinées à exercer l'une sur l'autre une force magnétique attractive.

La hauteur h de la protubérance 143 est suffisante pour pouvoir exercer une pression sur la pièce 123, ce qui conduit à la déformer, lorsque la fiche 140 coopère avec l'embase 120. La pièce 123 ainsi déformée établit un contact électrique avec les deux pièces 124 et 125.

L'embase 120 joue un double rôle :
- un rôle de connecteur électrique : puisqu'elle permet de connecter un dispositif (par exemple un patch générateur d'impulsions pour l'électrostimulation) à une électrode cutanée, via la fiche 140 et le câble électrique 33, plus précisément grâce au contact électrique établi entre la pièce 142 de la fiche 140 et la pièce 121 de l'embase 120 ;
- un rôle d'interrupteur : en coopération avec la fiche 140, puisque la protubérance 143 de la fiche 140 permet de relier électriquement la pièce 124 à la pièce 125, par l'intermédiaire de la pièce 123 lorsque la fiche 140 est connectée à l'embase 120, les deux pièces 124 et 125 étant sinon isolées électriquement.

On comprend que ce terme d'interrupteur ne correspond pas ici à celui d'interrupteur marche/arrêt permettant d'allumer et d'éteindre le dispositif d'électrosimulation. Cette fonction marche/arrêt est remplie par d'autres moyens qui ne sont pas décrits ici.

Les pièces 124 et 125 de l'embase sont reliées électriquement par l'intermédiaire de la protubérance 143 lorsque la fiche 140 est connectée à l'embase. Ceci permet donc aux circuits électroniques du patch 130 de reconnaître si une électrode amovible est ou non connectée au patch selon que l'interrupteur est ouvert ou fermé.

Il convient de noter que la pièce déformable 123 présente l'avantage d'éviter tout risque d'encrassement des pièces 124 et 125 au cours de la vie du dispositif.

Le connecteur électrique décrit aux figures 1 et 2 présente les avantages suivants :
1) Un actionnement accidentel de l'interrupteur, par exemple avec les doigts, est peu probable, car les pièces 124 et 125 sont positionnées au fond d'une cavité et non pas en surface de l'embase.
2) Un encrassement des contacts électriques, par exemple par de la poussière, est peu probable, car les pièces de l'embase ayant une fonction électrique sont :
   a. soit en surface de i'embase donc facilement nettoyables (pièce 121),
   b. soit au fond d'une cavité mais protégées contre l'encrassement (les pièces 124 et 125 sont protégées par la pièce 123),

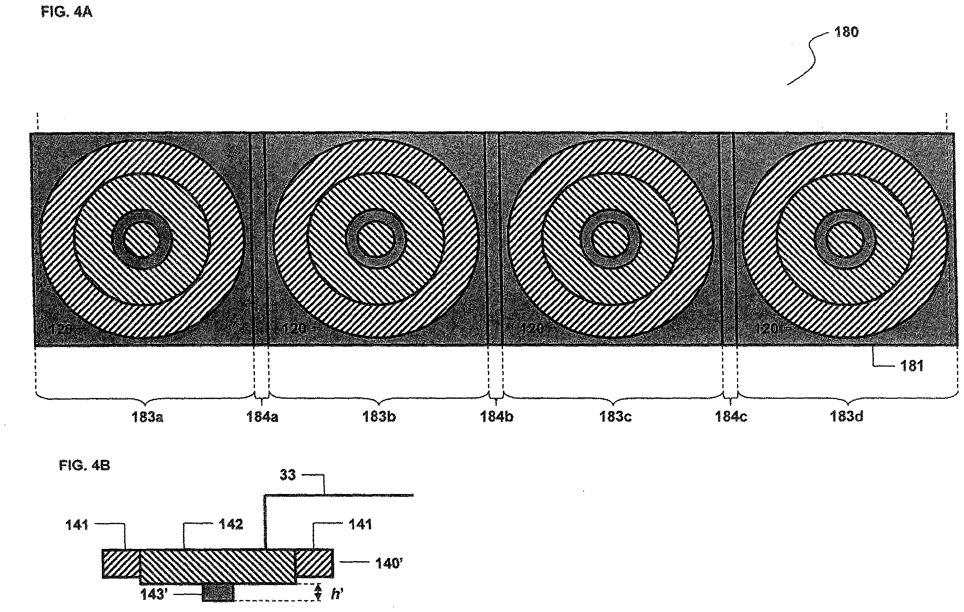

La figure 4A représente (vu de dessus) un patch générateur d'impulsions pour l'électrostimulation 180.

Le patch 180 comprend une partie principale 181 qui contient notamment des composants électroniques et au moins une source d'énergie électrique.

Le patch 180 comprend sur sa face supérieure quatre embases 120 décrites à la figure 1.

Le patch 180 peut être subdivisé en sept zones :
- quatre zones rigides 183a, 183b, 183c, 183d : chacune de ces quatre zones rigides comprend une embase 120 et un ensemble de composants (composants électroniques, sources d'énergie électrique, etc.). De préférence, dans chacune de ces quatre zones rigides, l'embase 120 est alignée verticalement avec l'ensemble de composants, de manière à le recouvrir et donc à assurer sa protection mécanique ;
- trois zones flexibles 184a, 184b, 184c: comprenant des pistes métalliques permettant d'interconnecter les composants positionnés dans les zones rigides 183a, 183b, 183c, 183d.

Le fait de disposer d'une architecture composée de zones rigides (183a, 183b, 183c, 183d) reliées par des zones flexibles (184a, 184b, 184c) permet d'assurer la flexibilité et la conformabilité du patch 180 dans son ensemble.

Au sein de chacune des quatre zones rigides 183a, 183b, 183c et 183d, le fait de combiner, dans un même élément 120, la double fonction de moyen de connexion électrique et de protection mécanique permet de gagner en compacité et en finesse, tout en assurant la fiabilité et la robustesse du patch 180.

Les embases 120 du patch 180 peuvent coopérer avec deux types de fiches : des fiches du type 140 décrites à la figure 3 et des fiches du type 140' décrites à la figure 4B.

La seule différence entre une fiche 140 (figure 3) et une fiche 140' (figure 4B) se situe au niveau de la pièce cylindrique centrale formant une protubérance en saillie par rapport aux pièces 141 et 142.

Dans la fiche 140', la pièce 143 de hauteur h formant une protubérance est remplacée par une pièce 143' de hauteur h' formant une protubérance, avec h' < h.

La connexion d'une fiche 140 dans une embase 120 permet à la fois :
- d'établir un contact électrique entre la pièce 142 de la fiche 140 et la pièce 121 de l'embase 120 (rôle de connecteur électrique)
- et de relier électriquement la pièce 124 à la pièce 125 (rôle d'interrupteur en permettant le passage de courant entre les deux pièces 124 et 125).

Par contre, la connexion d'une fiche 140' dans une embase 120 :
- permet d'établir un contact électrique entre la pièce 142 de la fiche 140' et la pièce 121 de l'embase 120 (rôle de connecteur électrique)
- mais ne permet pas de relier électriquement la pièce 124 à la pièce 125 (pas de rôle d'interrupteur car les deux pièces 124 et 125 restent isolées électriquement), car la hauteur h' de la pièce 143' de la fiche 140' n'est pas suffisante pour déformer la pièce 123.

On comprend donc que les circuits électroniques du patch 180 peuvent ainsi reconnaître automatiquement quel est le type d'électrodes connectées au patch.

En particulier, les circuits électroniques du patch 180 sont capables de reconnaître si une embase 120 est connectée à une fiche 140 ou à une fiche 140'. Pour cela, les circuits électroniques du patch 180 observent l'état (ouvert ou fermé) de l'interrupteur associé à l'embase 120 (c'est-à-dire l'interrupteur constitué des pièces 124 et 125). Par exemple, les circuits électroniques du patch 180 peuvent comprendre un microcontrôleur, dont une entrée numérique est connectée à une résistance de type « pull-up » afin d'observer l'état (ouvert ou fermé) de l'interrupteur. Le principe de fonctionnement d'une résistance « pull-up » est bien connu dans l'état de l'art : l'entrée du microcontrôleur est à l'état logique 1 lorsque l'interrupteur est ouvert, et à l'état logique 0 lorsque l'interrupteur est fermé. Par conséquent, dans cet exemple, l'entrée du microcontrôleur est à l'état logique 1 lorsqu'une fiche 140' est connectée à l'embase 120, et à l'état logique 0 lorsqu'une fiche 140 est connectée à l'embase 120.

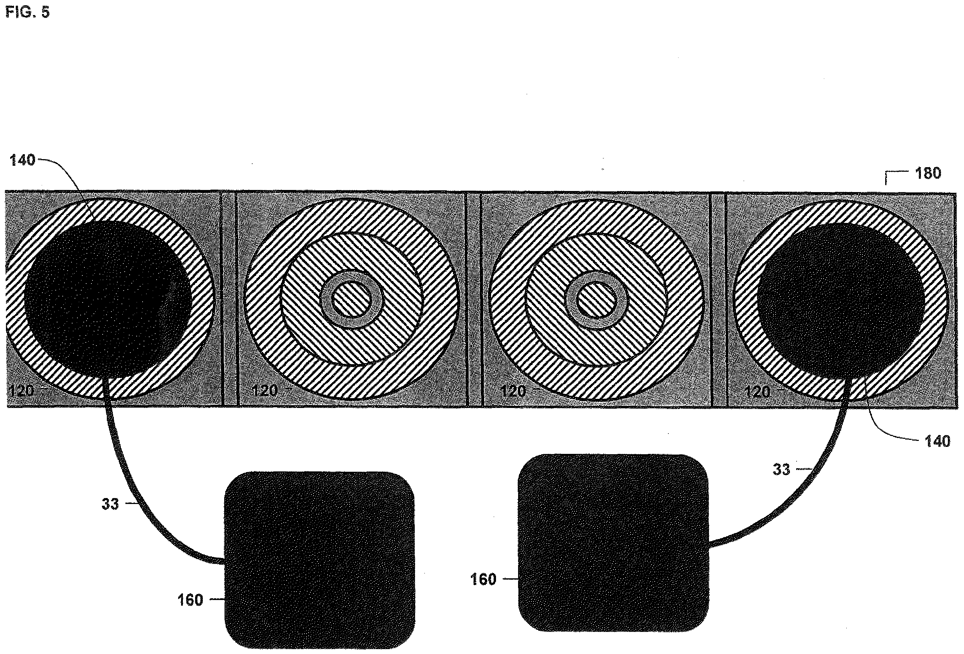

Dans une première utilisation du patch 180, illustrée à la figure 5, l'utilisateur connecte un seul couple d'électrodes cutanées 160 au patch 180.

Chacune des deux électrodes cutanée 160 est reliée à un câble électrique 33, lui-même solidaire d'une fiche 140 qui est connectée à une embase 120.

Il est important de noter que chacune des deux fiches 140 peut être connectée dans n'importe laquelle des quatre embases 120, étant donné que les quatre embases 120 sont identiques et peuvent toutes coopérer avec une fiche 140.

Dans le cas décrit à la figure 5, le patch 180, qui comprend quatre embases 120, n'est relié qu'à deux électrodes cutanées 160. Le fait de disposer d'un nombre de moyens de connexion 120 (quatre dans cet exemple) supérieur au nombre de fiches à connecter (deux dans cet exemple) est avantageux, car cela offre à l'utilisateur une grande liberté de positionnement des électrodes cutanées 160 sur le corps, pour une position du patch 180 donnée.

Ainsi, l'utilisateur dispose d'une grande liberté de positionnement des électrodes cutanées, et donc une grande liberté d'ajustement de la distance inter-électrodes.

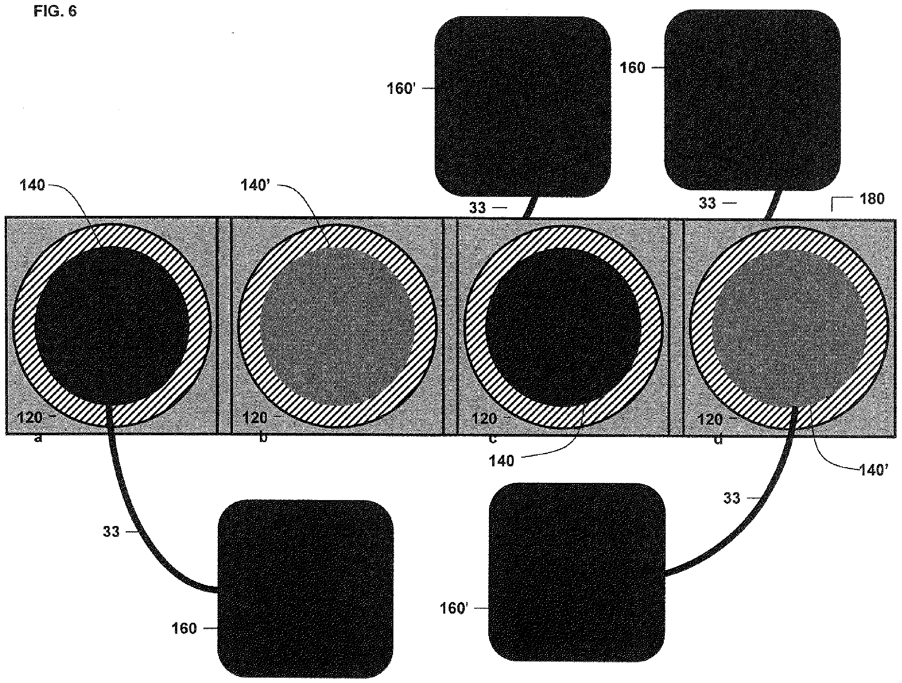

Dans une deuxième utilisation du patch 180, illustrée à la figure 6, l'utilisateur connecte deux couples d'électrodes cutanées 160, 160' au patch 180 (soit quatre électrodes). Ceci permet d'effectuer simultanément deux programmes d'électrostimulation, chaque programme correspondant à un des couples. Ces deux programmes peuvent être différents, il est donc important que les circuits électroniques du patch 180 puissent reconnaître si une électrode cutanée appartient au premier couple (électrode cutanée 160) ou au deuxième couple (électrode cutanée 160').

Chacune des électrodes cutanées 160 du premier couple est reliée à un câble électrique 33, lui-même solidaire d'une fiche 140 qui est connectée à une embase 120. Chacune des électrodes cutanées 160' du deuxième couple est reliée à un câble électrique 33, lui-même solidaire d'une fiche 140' qui est connectée à une embase 120.

Les circuits électroniques du patch 180 peuvent alors reconnaître si une électrode cutanée appartient au premier couple ou au deuxième couple :
- une électrode cutanée 160 du premier couple est solidaire d'une fiche 140, dont la connexion à une embase 120 entraîne la fermeture de l'interrupteur comprenant les pièces 124 et 125,
- une électrode cutanée 160' du deuxième couple est solidaire d'une fiche 140', dont la connexion à une embase 120 n'entraîne pas la fermeture de l'interrupteur comprenant les pièces 124 et 125.

Il est important de remarquer que chacune des fiches 140 ou 140' peut être connectée dans n'importe laquelle des quatre embases 120, étant donné que les quatre embases 120 sont identiques et peuvent coopérer avec une fiche 140 ou 140'.

Cela offre à l'utilisateur une grande liberté de positionnement des électrodes cutanées 160 et 160' sur le corps, pour une position du patch 180 donnée, et donc une grande liberté d'ajustement de la distance inter-électrodes.

Par ailleurs, la reconnaissance d'une électrode peut être réalisée indépendamment de l'embase dans laquelle elle est connectée.

D'autres applications de la fonction interrupteur sont envisageables, notamment la reconnaissance automatique des embases dans lesquelles une fiche a été connectée.

Dans le cas par exemple d'un patch possédant quatre embases identiques, permettant de connecter le patch à un couple d'électrodes cutanées ou à deux couples d'électrodes cutanées, les embases dans lesquelles une fiche a été connectée ont leur interrupteur dans un état fermé, alors que les embases dans lesquelles aucune fiche n'a été connectée ont leur interrupteur dans un état ouvert. Ainsi, lorsque le circuit électronique est allumé, il peut détecter l'état ouvert ou fermé de l'interrupteur intégré dans une embase pour en tirer des informations sur la connexion ou la non-connexion d'une électrode dans cette embase. L'électronique du patch peut alors distinguer le cas d'une situation normale (deux fiches connectées ou quatre fiches connectées), du cas d'une situation anormale (aucune fiche connectée, ou une seule fiche connectée, ou trois fiches connectées).

Dans le cas d'une situation anormale, le patch bloque le lancement de programmes d'électrostimulation afin de protéger l'utilisateur.

Dans le cas d'une situation normale où deux fiches sont connectées (c'est-à-dire où un seul couple d'électrodes cutanées est connecté), le patch autorise le lancement de programmes d'électrostimulation sur ce couple d'électrodes cutanées. Cependant, le patch ne met pas sous tension les deux embases laissées inoccupées, toujours afin de protéger l'utilisateur.

Dans le cas d'une situation normale où quatre fiches sont connectées (c'est-à-dire où deux couples d'électrodes cutanées sont connectées), le patch autorise le lancement de programmes d'électrostimulation sur les deux couples d'électrodes cutanées.

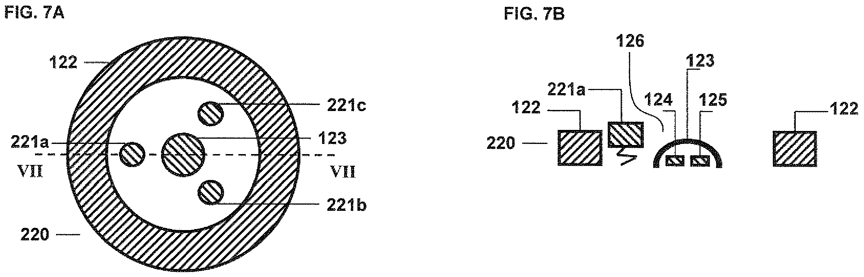

Une variante de l'embase 120 est décrite en référence à la figure 7.

La figure 7A (vue de dessus) et la figure 7B (vue en coupe selon la ligne VII-VII sur la figure 7A) représentent une embase 220, qui est une variante de l'embase 120.

Dans l'embase 220, trois pièces 221a, 221b et 221c remplacent la pièce 121 de l'embase 120. Ces trois pièces 221a, 221b et 221c sont disposées selon un cercle concentrique avec la pièce annulaire 122 et de diamètre inférieur. La pièce 123 est située à l'intérieur de ce cercle.

Comme la pièce 121 de l'embase 120, les trois pièces 221a, 221b et 221c de l'embase 220 ont une fonction électrique, et sont destinées à établir un contact électrique avec la pièce 142 d'une fiche 140 ou 140'.

Les trois pièces 221a, 221b et 221c sont avantageusement montées sur ressort, ce qui autorise une tolérance plus grande sur les dimensions des différentes pièces constituant l'embase et les fiches.

L'invention n'est cependant pas limitée à ce mode de réalisation. En pratique, il suffit de prévoir une seule de ces pièces. Par ailleurs, plus de trois pièces pourraient être prévues.

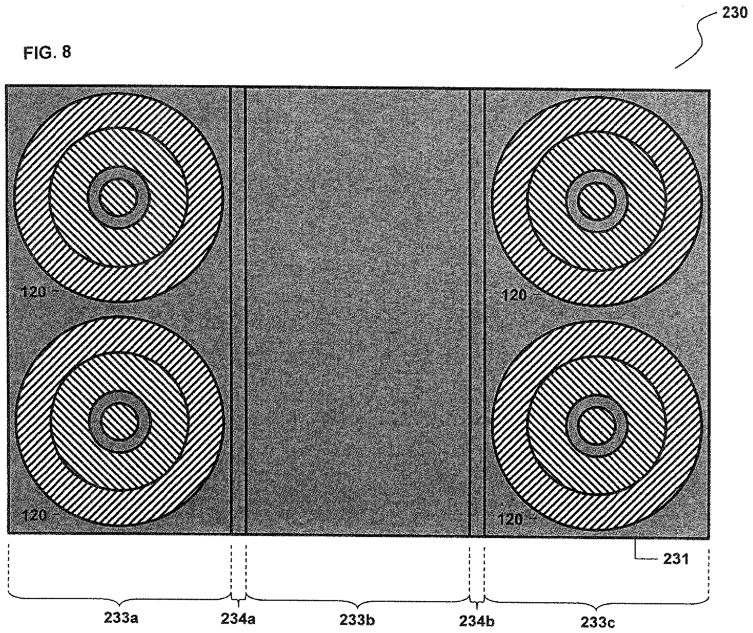

Une variante du patch générateur d'impulsions 180 est décrit en référence à la figure 8 qui représente un patch générateur d'impulsions 230.

Le patch 230 comprend une partie principale 231 qui contient notamment des composants électroniques et au moins une source d'énergie électrique.

Le patch 230 comprend sur sa face supérieure quatre embases 120 décrites à la figure 1.

Le patch 230 peut être subdivisé en cinq zones :
- trois zones rigides 233a, 233b, 233c: chacune de ces trois zones rigides comprend un ensemble de composants (composants électroniques, sources d'énergie électrique, etc.). Les zones 233a et 233c comprennent en plus chacune deux embases 120. De préférence, dans les zones 233a et 233c, les embases 120 sont alignées verticalement avec les composants, de manière à les recouvrir et donc à assurer leur protection mécanique ;
- deux zones flexibles 234a, 234b : comprenant des pistes métalliques permettant d'interconnecter les composants positionnés dans les zones rigides 233a, 233b, 233c.

Par rapport au patch 180, le patch 230 est donc moins long, ce qui est avantageux pour un positionnement sur certaines zones du corps (par exemple les avant-bras).

Les signes de référence insérés après les caractéristiques techniques figurant dans les revendications ont pour seul but de faciliter la compréhension de ces dernières et ne sauraient en limiter la portée.

## Revendications

1. Dispositif médical cutané, comprenant :
- une partie principale (131, 181, 231) contenant une source d'énergie électrique (40) susceptible de générer un courant électrique et des composants électroniques, pour former partie d'un circuit électrique,
- au moins une électrode cutanée amovible comportant une fiche (140, 140') de connexion électrique, ladite électrode étant destinée à être en contact électrique avec l'utilisateur ;
- au moins une embase (120) du type femelle, c'est-à-dire comportant un trou (126), ladite embase (120) étant positionnée sur une première face de la partie principale et susceptible d'être connectée électriquement avec ladite au moins une électrode cutanée amovible; **caractérisé en ce que** ledit dispositif comprend, dans ladite au moins une embase, des moyens conducteurs (124, 125) et, dans ladite fiche, un moyen (143; 143') de contrôle du passage du courant entre lesdits moyens conducteurs, **en ce que** les moyens conducteurs (124, 125) sont protégés par une pièce (123) conductrice déformable commune en forme de coupole et **en ce que** lesdits moyens conducteurs et ledit moyen de contrôle forment un interrupteur électrique ayant une fonction de reconnaissance de ladite au moins une électrode cutanée amovible en connectant électriquement les moyens conducteurs (124, 125) via une déformation de ladite pièce (123) conductrice.

2. Dispositif selon la revendication 1, dans lequel l'embase (120) comporte au moins une pièce (121 ; 221a à 221c) ayant une fonction électrique et la fiche (140, 140') comporte une pièce cylindrique (142) ayant une fonction électrique, la connexion électrique entre l'embase et la fiche étant assurée par le contact électrique entre les pièces (121 ; 221a à 221c ; 142) ayant une fonction électrique.

3. Dispositif selon la revendication 2, dans lequel une pièce annulaire (121) a une fonction électrique.

4. Dispositif selon la revendication 2, dans lequel trois pièces (221a à 221c) disposées selon un cercle ont une fonction électrique.

5. Dispositif selon la revendication 4, dans lequel lesdites pièces sont montées sur ressort.

6. Dispositif selon l'une des revendications 1 à 5, dans lequel lesdits moyens conducteurs de l'embase comprennent deux ensembles (124, 125) fixes et séparés l'un de l'autre par une zone isolante.

7. Dispositif selon la revendication 6, dans lequel la fiche (140) de ladite au moins une électrode amovible (160) comporte une protubérance (143) présentant une hauteur (h) telle que la protubérance (143) est susceptible de venir en contact avec les deux ensembles (124, 125) fixes.

8. Dispositif selon la revendication 6, dans lequel la fiche (140') de ladite au moins une électrode amovible (160') comporte une protubérance (143') présentant une hauteur (h') telle qu'un écartement existe entre elle et les deux ensembles fixes (124, 125), lorsqu'elle est insérée dans le trou (126).

9. Dispositif selon la revendication 6, dans lequel la fiche (140) de ladite au moins une électrode amovible (160) comporte une protubérance (143) présentant une hauteur (h) telle que la protubérance (143) est susceptible de déformer ladite pièce (123) lorsqu'elle est insérée dans le trou (126).

10. Dispositif selon la revendication 6, dans lequel la fiche (140') de ladite au moins une électrode amovible (160') comporte une protubérance (143') présentant une hauteur (h') telle que la pression exercée par la protubérance (143') sur ladite pièce (123) est nulle, lorsqu'elle est insérée dans le trou (126).

11. Dispositif selon l'une des revendications 1 à 10, comprenant au moins deux embases (120) et au moins deux électrodes cutanées amovibles (160, 160').

12. Dispositif selon l'une des revendications 1 à 11, comprenant au moins deux zones rigides (183a à 183d ; 233a à 233c) et une zone flexible (184a à 184c ; 234a, 234b) entre deux zones rigides adjacentes.

13. Dispositif selon l'une des revendications 1 à 12, dans lequel ladite partie principale (131, 181, 231) comporte sur une deuxième face, opposée à la première, une autre électrode cutanée (132) destinée à établir un contact électrique avec l'utilisateur.

14. Dispositif selon l'une des revendications 1 à 13, dans lequel sont prévus des moyens magnétiques à la fois sur la fiche et sur l'embase.

## Patentansprüche

1. Kutane medizinische Vorrichtung, umfassend:
- einen Hauptteil (131, 181, 231), der eine Quelle elektrischer Energie (40), die imstande ist, einen elektrischen Strom zu erzeugen, und Elektronikkomponenten enthält, um Teil eines elektrischen Stromkreises zu bilden,
- mindestens eine abnehmbare kutane Elektrode, die einen Stecker (140, 140') zur elektrischen Verbindung umfasst, wobei die Elektrode dazu bestimmt ist, mit dem Benutzer in elektrischem Kontakt zu stehen;
- mindestens eine Basisplatte (120) vom Buchsentyp, das heißt eine Öffnung (126) umfassend, wobei die Basisplatte (120) auf einer ersten Seite des Hauptteils positioniert ist und imstande ist, mit der mindestens einen abnehmbaren kutanen Elektrode elektrisch verbunden zu werden;
**dadurch gekennzeichnet, dass** die Vorrichtung in der mindestens einen Basisplatte leitfähige Mittel (124, 125) und in dem Stecker ein Mittel (143; 143') zum Steuern des Stromdurchgangs zwischen den leitfähigen Mitteln umfasst, dadurch, dass die leitfähigen Mittel (124, 125) durch ein gemeinsames verformbares leitfähiges Bauteil (123) in Kuppelform geschützt sind und dadurch, dass die leitfähigen Mittel und das Steuermittel einen elektrischen Schalter bilden, der eine Erkennungsfunktion der mindestens einen abnehmbaren kutanen Elektrode aufweist, indem die leitfähigen Mittel (124, 125) über eine Verformung des leitfähigen Bauteils (123) elektrisch verbunden werden.

2. Vorrichtung nach Anspruch 1, wobei die Basisplatte (120) mindestens ein Bauteil (121; 221a bis 221c) umfasst, das eine elektrische Funktion aufweist, und der Stecker (140, 140') ein zylindrisches Bauteil (142) umfasst, das eine elektrische Funktion aufweist, wobei die elektrische Verbindung zwischen der Basisplatte und dem Stecker durch den elektrischen Kontakt zwischen den Bauteilen (121; 221a bis 221c; 142), die eine elektrische Funktion aufweisen, gewährleistet ist.

3. Vorrichtung nach Anspruch 2, wobei ein ringförmiges Bauteil (121) eine elektrische Funktion aufweist.

4. Vorrichtung nach Anspruch 2, wobei drei Bauteile (221a bis 221c), die in einem Kreis angeordnet sind, eine elektrische Funktion aufweisen.

5. Vorrichtung nach Anspruch 4, wobei die Bauteile federmontiert sind.

6. Vorrichtung nach einem der Ansprüche 1 bis 5, wobei die leitfähigen Mittel der Basisplatte zwei Anordnungen (124, 125) umfassen, die fest angebracht sind und durch einen isolierenden Bereich voneinander getrennt sind.

7. Vorrichtung nach Anspruch 6, wobei der Stecker (140) der mindestens einen abnehmbaren Elektrode (160) einen Vorsprung (143) umfasst, der eine Höhe (h) derart aufweist, dass der Vorsprung (143) imstande ist, mit den zwei fest angebrachten Anordnungen (124, 125) in Kontakt zu kommen.

8. Vorrichtung nach Anspruch 6, wobei der Stecker (140') der mindestens einen abnehmbaren Elektrode (160') einen Vorsprung (143') umfasst, der eine Höhe (h') derart aufweist, dass ein Abstand zwischen dieser und den zwei fest angebrachten Anordnungen (124, 125) besteht, wenn sie in die Öffnung (126) eingesetzt wird.

9. Vorrichtung nach Anspruch 6, wobei der Stecker (140) der mindestens einen abnehmbaren Elektrode (160) einen Vorsprung (143) umfasst, der eine Höhe (h) derart aufweist, dass der Vorsprung (143) imstande ist, das Bauteil (123) zu verformen, wenn sie in die Öffnung (126) eingesetzt wird.

10. Vorrichtung nach Anspruch 6, wobei der Stecker (140') der mindestens einen abnehmbaren Elektrode (160') einen Vorsprung (143') umfasst, der eine Höhe (h') derart aufweist, dass der von dem Vorsprung (143') auf das Bauteil (123) ausgeübte Druck null ist, wenn sie in die Öffnung (126) eingesetzt wird.

11. Vorrichtung nach einem der Ansprüche 1 bis 10, umfassend mindestens zwei Basisplatten (120) und mindestens zwei abnehmbare kutane Elektroden (160, 160').

12. Vorrichtung nach einem der Ansprüche 1 bis 11, umfassend mindestens zwei steife Bereiche (183a bis 183d; 233a bis 233c) und einen flexiblen Bereich (184a bis 184c; 234a, 234b) zwischen zwei angrenzenden steifen Bereichen.

13. Vorrichtung nach einem der Ansprüche 1 bis 12, wobei der Hauptteil (131, 181, 231) auf einer zweiten Seite, die der ersten gegenüberliegt, eine weitere kutane Elektrode (132) umfasst, die dazu bestimmt ist, mit dem Benutzer einen elektrischen Kontakt herzustellen.

14. Vorrichtung nach einem der Ansprüche 1 bis 13, wobei Magnetmittel zugleich auf dem Stecker und auf der Basisplatte vorgesehen sind.

## Claims

1. A cutaneous medical device, comprising:
- a main part (131, 181, 231) containing an electricity source (40) capable of generating an electric current and electronic components, to form part of an electric circuit,
- at least one removable cutaneous electrode including a plug (140, 140'), said electrode being intended to be in electrical contact with the user,
- at least one base (120) positioned on a first face of the main part and able to be electrically connected with said removable cutaneous electrode;
**characterized in that** said device comprises, in said at least one base, conductive means (124, 125) and, in said plug, a means (143; 143') for controlling the passage of the current between said conductive means, **in that** the conductive means (124, 125) are housed by a common and deformable conductive piece (123) in the shape of a cupola and **in that** said conductive means and said control means form an electric switch having a recognition function for said removable cutaneous electrode by electrically connecting the conductive means (124, 125) via a deformation of said conductive piece (123).

2. The device according to claim 1, wherein the base (120) includes at least one part (121; 221a to 221c) having an electrical function and the plug (140, 140') includes a cylindrical part (142) having an electrical function, the electrical connection between the base and the plug being provided by the electrical contact between the parts (121; 221a to 221c; 142) having an electrical function.

3. The device according to claim 2, wherein an annular part (121) has an electrical function.

4. The device according to claim 2, wherein three parts (221a to 221c) arranged in a circle have an electrical function.

5. The device according to claim 4, wherein said parts are mounted on a spring.

6. The device according to one of claims 1 to 5, wherein said conductive means of the base comprise two assemblies (124, 125) that are fixed and separated from one another by an insulating area.

7. The device according to claim 6, wherein the plug (140) of said at least one removable electrode (160) includes a protuberance (143) having a height (h) such that the protuberance (143) is able to come into contact with both fixed assemblies (124, 125).

8. The device according to claim 6, wherein the plug (140') of said at least one removable electrode (160') includes a protuberance (143') having a height (h') such that a gap exists between it and the two fixed assemblies (124, 125), when it is inserted into the cavity (126).

9. The device according to claim 6, wherein the plug (140) of said at least one removable electrode (160) includes a protuberance (143) having a height (h) such that the protuberance (143) is able to deform said part (123) when it is inserted in the cavity (126).

10. The device according to claim 6, wherein the plug (140') of said at least one removable electrode (160') includes a protuberance (143') having a height (h') such that the pressure exerted by the protuberance (143') on said part (123) is zero, when it is inserted into the cavity (126).

11. The device according to one of claims 1 to 10, comprising at least two bases (120) and at least two removable cutaneous electrodes (160, 160').

12. The device according to one of claims 1 to 11, comprising at least two rigid zones (183a to 183d; 233a to 233c) and one flexible zone (184a to 184c; 234a, 234b) between two adjacent rigid zones.

13. The device according to one of claims 1 to 12, wherein said main part (131, 181, 231) includes, on a second face, opposite the first, another cutaneous electrode (132) intended to establish electrical contact with the user.

14. The device according to one of claims 1 to 13, wherein magnetic means are provided both on the plug and the base.
